# EUROPÄISCHE PATENTSCHRIFT

(11) **EP 1 909 857 B1**
(45) Veröffentlichungstag und Bekanntmachung des Hinweises auf die Patenterteilung: **14.09.2011**
(21) Anmeldenummer: 05823938.5
(22) Anmeldetag: 23.12.2005
(51) Int. Cl.: A61L 15/50, C08J 3/24

(54) **SUPERABSORBER, DAMIT AUSGERÜSTETE NANOFASERVLIESE UND DEREN VERWENDUNG**
SUPERABSORBENTS, NANOFIBRE WEBS FINISHED THEREWITH, AND USE THEREOF
SUPERABSORBANT, NON TISSES A BASE DE NANOFIBRES AYANT SUBI UN FINISSAGE AU MOYEN DE CE DERNIER ET LEUR UTILISATION

(30) Priorität: 05.08.2005 DE 102005036992; 16.11.2005 DE 102005054698
(43) Veröffentlichungstag der Anmeldung: 16.04.2008
(60) Teilanmeldung: 11002242.3
(73) Patentinhaber: Schill + Seilacher GmbH, 71032 Böblingen (DE)
(72) Erfinder: RING, Horst, 71034 Böblingen (DE); HARBIG, Roland, 71032 Böblingen (DE)
(74) Vertreter: Prinz & Partner
(86) Internationale Anmeldenummer: PCT/EP2005/014017
(87) Internationale Veröffentlichungsnummer: WO 2007/016970

(56) Entgegenhaltungen:
- US-A1- 2004 176 557
- US-A1- 2004 186 244

## Beschreibung

Die Erfindung betrifft ein Superabsorberpulver, bestehend aus Polymerpartikeln, die einen in Gegenwart von Wasser aufquellenden Kern und eine oberflächlich nachvernetzte Schale aufweisen, dessen Verwendung zur Ausrüstung von textilen Flächengebilden aus Feinstfasern oder -filamenten mit einem Durchmesser von weniger als 10 µm sowie die Verwendung eines mit dem Superabsorber zur Absorption und Retention hydrophiler Fluide ausgerüsteten Nanofaservlieses zur Aufnahme und/oder Abgabe eines oder mehrerer Fluide oder Fluidgemische.

Unter "Superabsorbern" werden polymere Materialien verstanden, die bis zum Tausendfachen ihrer Masse an Wasser oder anderen Fluiden aufnehmen können, wobei sie unter Bildung eines Gels aufquellen. Da das Aufnahme- und Retentionsvermögen der Superabsorber durch den "Gelblocking"-Effekt, das Verklumpen der an- oder aufgequollenen Superabsorberteilchen, schnell stark begrenzt wird, hat man bereits verschiedene Verfahren zur oberflächlichen Nachvernetzung der Superabsorberteilchen vorgeschlagen, die zu einem von innen nach außen ansteigenden Gradienten des Vernetzungsgrades der Teilchen, d.h. einem weniger vernetzten Kern und einer stärker vernetzten Schale, führen. Solche modifizierten Superabsorber und Verfahren zu ihrer Herstellung sind aus Ullmanns Encyclopedia of Industrial Chemistry, 6th Ed., Vol. 35, pp. 73 ff:, 2003, ebenso bekannt wie deren Verwendung zur Ausrüstung von Hygieneprodukten, worunter Windeln und andere Inkontinenzprodukte, Damenbinden und Wundverbände zu verstehen sind.

Studien zufolge leiden in Westeuropa ca. 15 Millionen und in den USA etwa dieselbe Anzahl Menschen an Urininkontinenz. Diese Gesundheitsstörung tritt bei Frauen 10 Mal häufiger auf als bei Männern. Ca. 25 % Frauen im Alter von 30 - 59 Jahren sind zumindest vorübergehend inkontinent. Aber auch 10 - 20% der jüngeren Frauen im Alter um die 20 Jahre sind betroffen. In Westeuropa werden jährlich über 5,2 Milliarden Stück Inkontinenzprodukte wie z. B. Erwachsenenwindeln verbraucht. In USA werden die jährlichen Ausgaben für Inkontinenzprodukte auf über 16,4 Milliarden US Dollar beziffert, wovon ca. 35 % in Pflegeheimen anfallen. Hieraus ergibt sich die große soziale und wirtschaftliche Bedeutung der Entwicklung verbesserter Superabsorber, die speziell an die für solche Inkontinenzprodukte verwendeten textilen Flächengebilde, meist Faservliese, angepaßt sind.

Inkontinenzprodukte haben in erster Linie die Aufgabe, Urin aufzunehmen, fest zu absorbieren und effektiv zu binden. Dabei soll Nässe von der Haut ferngehalten und Geruchsbildung unterdrückt werden. Das wird durch einen mehrlagigen Aufbau aus Folie als Flüssigkeitssperre nach außen, verschiedenen Vliesstoffen zur Steuerung der Flüssigkeitsverteilung und Superabsorber zur sicheren Flüssigkeitsabsorption erzielt.

Neben dieser technischen Funktionalität ist ein gewisser Tragekomfort, gute Paßform, Diskretion (Vertriebswege, Volumen, Knistern/Rascheln) und eine hygienische Handhabbarkeit erwünscht.

Inkontinenzprodukte ermöglichen bereits heute geringer betroffenen Personen einen weitgehend normalen Lebensalltag. Dennoch ist die Mobilität eingeschränkt und der Aufenthalt auf eine Umgebung mit einer sanitären Infrastruktur begrenzt, was nicht zuletzt auch die soziale und berufliche Situation beeinflußt.

Im Falle stärkerer Inkontinenz treten diese Beeinträchtigungen besonders hervor, da es mit heutigen Materialien und Konstruktionen von Inkontinenzprodukten zwar gelingt, die technischen Probleme zu beherrschen, jedoch zu Lasten von Tragekomfort, Paßform, Diskretion und Handhabung.

Da Inkontinenz ein gesellschaftliches Tabuthema ist, hat die sozialpsychologische Komponente dieser Gesundheitsstörung insbesondere bei jüngeren Frauen eine besondere Bedeutung, die bis hin zu Isolation, Vereinsamung und Verlust von Selbstbewußtsein und Autonomie führen kann.

Hier können neuartige Materialien für den Einsatz in neuartigen Inkontinenzprodukten Abhilfe schaffen, die insbesondere dem Bedürfnis nach Diskretion durch geringes Volumen, geringe Dicke, Geräuscharmut und hohen Tragekomfort Rechnung tragen.

Aus dem US-Patent 5629377 ist es bereits bekannt, Superabsorberpulver aus oberflächlich nachvernetzten Polymerpartikeln zur Ausrüstung von Windeln und anderen Inkontinenzprodukten zu verwenden. Es hat sich jedoch gezeigt, daß kommerziell verfügbare Superabsorberpulver in Kombination mit Feinstfaservliesen, die aus Gründen einer möglichst trockenen Hautoberfläche der Benutzer dieser Produkte bevorzugt werden, noch nicht optimal sind. Sie ließen sich nicht problemlos verarbeiten, waren nicht einwandfrei in die Vliesstruktur eingebunden und quollen bei Benetzung aus der Vliesstruktur heraus.

Die US 2004/0186244 A beschreibt ein Verfahren zur Herstellung von Hydrogelen, bei dem Acrylsäure teilweise neutralisiert und in Gegenwart eines Vernetzers polymerisiert wird. Das so erhaltene Produkt wird nach Trocknung und Zerkleinerung gesiebt und eine Siebfraktion mit Partikelgrößen < 850 µm anschließend oberflächlich nachvernetzt. Die erhaltenen oberflächlich nachvernetzten Partikel werden erneut unter Verwendung eines Siebs mit einer Maschenweite von 850 µm gesiebt.

Aus der US 2004/0176557 A1 ist ein Hydrogelpulver bekannt, welches durch Polymerisation von Acrylaten in Gegenwart eines Vernetzers mit anschließender Trocknung, Zerkleinerung und Abtrennung der Siebfraktion von 150 bis 850 µm hergestellt wird. Diese Siebfraktion wird anschließend mit 1,3-Propandiol oberflächlich nachvernetzt. Eine Weiterbehandlung der oberflächlich nachvernetzten Partikel ist nicht beschrieben.

Unter "Nanofaservliesen" sind Vliese aus Textilfasern mit einem Durchmesser von weniger als 10 µm, vorzugsweise von weniger als 1 µm, zu verstehen. Nanofaservliese und Verfahren zu ihrer Herstellung sind beispielsweise bekannt aus dem US-Patent 4,043,331 und der Internationalen Patentanmeldung WO 01/27365. Die aus dem genannten Stand der Technik-bekannten Nanofaservliese waren zwar nicht mit Superabsorbern ausgerüstet, wurden aber ihrerseits zur Ausrüstung von Hygieneprodukten und Wundverbänden verwendet.

Der Erfindung liegt die Aufgabe zugrunde, ein speziell für die Kombination mit Feinstfaservliesen geeignetes Superabsorberpulver anzugeben, dessen Verwendung bei der Ausrüstung von textilen Flächengebilden wie z.B. Hygieneprodukten zu wirtschaftlicheren Herstellungsmöglichkeiten führt, sowie Anwendungsmöglichkeiten für die mit dem Superabsorber zur Absorption und Retention hydrophiler Fluide ausgerüsteten Feinstfaservliese anzugeben, bei denen sich die vorteilhaften Eigenschaften der so ausgerüsteten Faservliese in ökonomischer, ökologischer oder technischer Hinsicht besonders stark bemerkbar machen.

Diese Aufgabe wird erfindungsgemäß mit einem Superabsorberpulver gemäß Anspruch 1 gelöst.

Es hat sich nämlich überraschenderweise gezeigt, daß die herkömmlichen Superabsorberpulver auch dann, wenn sie an sich eine optimale Korngröße und eine geeignete Korngrößenverteilung für ein bestimmtes Feinstfaservlies gehabt hätten, deshalb versagten, weil sie im Verlauf ihrer Herstellung mehrfach, auch nach ihrer Nachvernetzung, zerkleinert worden sind, wodurch die Kern/Schale-Struktur der Partikel verletzt wurde und der Gelblocking-Effekt erneut einsetzte. Dadurch waren optimale Wasseraufnahme- und Wasserretentionswerte bei Feinstfaservliesen nicht erreichbar, obwohl bei "normalen" bzw. gröberen Faservliesen brauchbare Ergebnisse mit denselben herkömmlichen Superabsorberpulvern erzielt werden konnten. Erst als sorgfältig ausgesiebte, nicht zerkleinerte Partikel als Superabsorberpulver ausgewählt wurden und in Kombination mit Feinstfaservliesen verwendet wurden, stellten sich die gewünschten optimalen Wasseraufnahme- und Wasserretentionswerte ein. Dies gilt aber nicht nur für die Absorption und Retention von Wasser, sondern auch von anderen hydrophilen Fluiden.

Die verwendete Siebfraktion weist eine Korngrößenverteilung von d50 = 55 bis 100 µm und d100 = 100 bis 150 µm auf, womit erfindungsgemäß die besten Ergebnisse in Kombination mit Nanofaservliesen erziel werden. Die Angabe "d50 = 55 µm" bedeutet, daß 50 Gew.-% der Teilchen eine Korngröße von bis zu 55 µm, also 55 µm oder weniger, besitzen, und "d100 = 100µm" bedeutet, daß 100 Gew.-% der Teilchen eine Korngröße von bis zu 100 µm besitzen, also kein Teilchen größer als 100 µm ist.

Vorzugsweise ist das Polymer, aus dem die Superabsorberpartikel bestehen, ein (Meth)Acrylat oder (Meth)Acryl-Copolymerisat, besonders bevorzugt Natriumpolyacrylat.

Das Superabsorberpulver der ausgewählten Siebfraktion wird erfindungsgemäß zur Ausrüstung von textilen Flächengebilden aus Feinstfasern oder -filamenten mit einem Durchmesser von weniger als 10 µm verwendet. Vorzugsweise besitzen die Fasern oder Filamente einen Durchmesser von weniger als 1 µm. Solche Feinstfasern werden als Mikro- bzw. Nanofasern bezeichnet.

Die Verwendung der Superabsorberpulver zur Ausrüstung von Nanofaservliesen, insbesondere von elektrostatisch gesponnenen Nanofasem, hat sich als besonders vorteilhaft erwiesen, was weiter unten noch ausführlich erläutert werden wird.

Vorzugsweise bestehen die Fasern oder Filamente aus thermoplastischem und hydrophilem oder hydrophiliertem, schmelzspinnfähigem Polymerisat, wobei Polyurethan besonders bevorzugt ist.

Das erfindungsgemäße Superabsorberpulver wird vorzugsweise zur Ausrüstung von aus textilen Flächengebilden gebildeten Hygieneprodukten verwendet. Besonders bevorzugt werden erfindungsgemäß Windeln und andere Inkontinenzprodukte, Damenbinden und Wundverbände fluidaufsaugend ausgerüstet.

Ein bevorzugtes Ergebnis der erfindungsgemäßen Verwendung ist somit ein elastisches, superabsorbierendes Vlies aus Nanofasern für den Einsatz in neuartigen Hygieneartikeln, besonders in Inkontinenzprodukten mit verbessertem Tragekomfort, sicherer Geruchsbindung und erhöhter Diskretion. Der Hygieneartikel besteht aus einem Vliesstoff aus thermoplastischem Polyurethan, in das Superabsorberpulver aus Polyacrylat mit einer speziellen Korngrößenverteilung und einer speziellen Nachvernetzung mechanisch eingebunden ist. Der Anteil an speziellem Superabsorber kann bis zu 85 Gew.-% des Gesamtgewichts des ausgerüsteten Textils betragen.

Das mit dem erfindungsgemäßen Superabsorber zur Absorption und Retention hydrophiler Fluide ausgerüstete Nanofaservlies wird vorzugsweise zur Aufnahme und/oder verzögerten Abgabe mindestens eines der folgenden Fluide verwendet: Körperflüssigkeiten, Schweiß von Menschen und Tieren, Wasser, einschließlich Kühlwasser, Kondenswasser und Wasserdampf, Chemikalien, einschließlich Agrochemikalien und Pestiziden, Arzneimittel, Biozide, Germizide und Fungizide, Diagnostika, Brandschutz- und Feuerlöschmittel, Putz- und Reinigungsmittel, Hydraulikfluide, Heiz- und Kühlfluide, Abwässer, einschließlich radioaktiv kontaminierter Fluide, Parfümerien.

Zur Aufnahme und/oder verzögerten Abgabe von Körperflüssigkeiten wird das erfindungsgemäße Nanofaservlies vorzugsweise als Inkontinenzartikel, Babywindel, Damenbinde, Wundverband, Kühlumschlag, Hygienetuch, Kosmetikpad oder Betteinlage oder als Teil der vorgenannten Gegenstände verwendet.

Zur Aufnahme und/oder verzögerten Abgabe von Schweiß menschlichen oder tierischen Ursprungs wird das erfindungsgemäße Nanofaservlies vorzugsweise als schweißaufsaugendes Einlagematerial für Schuhe, Bekleidungsstücke, Kopfbedeckungen, Stirnbänder, Handschuhe, Polstermöbel, Fahrzeugsitze, Sättel, Bettwäsche, Bettdecken, Pferdedecken, Sportartikel oder Sportgeräte verwendet.

Zur Aufnahme und/oder verzögerten Abgabe von Wasser, einschließlich Kühlwasser, Kondenswasser und Wasserdampf, wird das erfindungsgemäße Nanofaservlies vorzugsweise als Wischtuch, Aufnehmer, Unfallschutzmatte, Isomatte, Zeltplane, Feuchttuch, Trockentuch, Poliertuch, Putztuch oder Fensterleder oder als Teil der vorgenannten Gegenstände verwendet.

Im Baubereich wird das erfindungsgemäße Nanofaservlies zur Aufnahme und/oder verzögerten Abgabe von Wasser, einschließlich Kühlwasser, Kondenswasser und Wasserdampf, vorzugsweise als Boden- oder Wandbelag, Parkettunterspannbahn, Dachunterspannbahn, Brandschutzmatte, Leckageschutzmatte, Matte zur Auskleidung von Feuchträumen, Zelten, Fahrzeugen, Tanks oder Behältern oder als Teil der vorgenannten Gegenstände verwendet.

Eine weitere bevorzugte Verwendung des Nanofaservlieses gemäß der Erfindung ist als Filtermaterial, Verpackungsmaterial, Ummantelung, Dämmstoff oder Dichtmaterial oder als Teil der vorgenannten Gegenstände möglich. Hierunter fallen als bevorzugte Verwendungen die Verpackung gefährlicher Güter, die Ummantelung von Rohren und Rohrleitungen, von Pipelines, von Elektrokabeln, einschließlich Kommunikationskabeln und Energiekabeln, und von sämtlichen Gegenständen, in denen Fluide der oben genannten Art gelagert oder transportiert werden, sich in Form von Kondenswasser und Wasserdampf bilden oder infolge von Leckagen oder Unfällen austreten können.

Zur Aufnahme und/oder verzögerten Abgabe von Chemikalien, einschließlich Agrochemikalien und Pestiziden, wird das erfindungsgemäße Nanofaservlies vorzugsweise als Geovlies, Drainagematte, Agrovlies oder als Vlies zur verzögerten Abgabe von Arzneimitteln, Chemikalien, Düngemitteln oder Pestiziden verwendet. Bei diesem Anwendungsbereich kann es sich um Vliese handeln, die in Gewächshäusern oder auf dem Boden über Feldern, Äckern und Plantagen großflächig ausgebreitet werden, um die genannten Wirkstoffe entweder aufzusaugen oder verzögert abzugeben. Umweltkatastrophen, bei denen große Mengen umweltschädlicher Fluide ausgetreten sind, z.B. infolge von Verkehrsunfällen, stellen ein bedeutendes Einsatzgebiet für die erfindungsgemäßen Nanofaservliese dar.

Bei den erfindungsgemäß vorgeschlagenen Verwendungsmöglichkeiten für die mit dem Superabsorber ausgerüsteten Nanofaservliese macht sich das hohe Wasser- bzw. Fluidretentionsvermögen und die hohe Geschwindigkeit der Wasser- bzw. Fluidaufnahme bei gleichzeitig besonders langsamer, verzögerter (Wieder-) Abgabe der aufgenommenen Fluidmengen vorteilhaft bemerkbar. Dadurch können verschüttete Fluide auch in großen Mengen sehr schnell aufgenommen und beseitigt werden.

Andererseits lassen sich die Vliese vorbeugend überall dort vorteilhaft einsetzen, wo Leckagen zu befürchten sind und Wasser, aber auch giftige, ätzende, sauere, basische oder umweltgefährdende Fluide aus Rohrleitungen, Behältern, Verpackungen, Tanks, Fahrzeugen, Industrieanlagen usw. austreten können; die austretenden Fluide werden dann sofort von den Vliesen, mit denen die Rohrleitungen etc. ummantelt oder verpackt oder ausgekleidet worden sind, aufgenommen und aufgrund einer sehr rasch einsetzenden Dochtwirkung aufgesogen.

Die Vorteile der Erfindung werden nachfolgend am Beispiel von Hygieneprodukten auf Basis von textilen Flächengebilden erläutert, sollen aber nicht einschränkend verstanden werden:

### 1. Verbesserte Diskretion

Der Grund für Dicke und Voluminosität von Inkontinenzprodukten liegt in erster Linie darin, daß Superabsorberpulver (SAP) bei Kontakt mit Wasser quillt und die Partikel untereinander verkleben, wodurch weitere Wasseraufnahme erschwert bzw. verhindert wird (Gelblocking). Um Gelblocking zu vermeiden, wurde SAP bisher immer in Mischung zusammen mit kurzen Cellulosefasern (Pulp) eingesetzt, die die Partikel voneinander getrennt halten sollen. Dabei kann der Anteil von SAP maximal 50 % betragen. Pulp hat eine sehr geringe Dichte, ist deshalb sehr voluminös und vergrößert beträchtlich das Volumen der für die Flüssigkeitsaufnahme benötigten SAP/Pulp-Menge.

Um diese Nachteile zu umgehen und auf Pulp verzichten zu können, dabei jedoch gleichzeitig eine große Oberfläche wie SAP selbst zu gewährleisten, wurden bereits verschiedene Versuche unternommen, den Superabsorber nicht als Pulver zu verwendet, sondern in anderer Aufmachung, z. B. als Schaum, als Faservlies oder in Form mit Superabsorber beschichteter Fasern und Vliese. Diese Konstrukte fanden aber keine Verwendung aufgrund gravierender Nachteile wie z. B. Festigkeitsverlust nach Benetzung, Sprödigkeit/Brüchigkeit im trockenen Zustand oder ausgeprägte Anisotropie des Benetzungsverhaltens.

Der neuartige Ansatz des erfindungsgemäßen Materials besteht nun darin, die einzelnen Partikel von Superabsorberpulver mit definierter Korngrößenverteilung in eine elastische Vliesstruktur so einzubinden, daß die Partikel voneinander räumlich getrennt sind, sich bei Kontakt mit Wasser vollflächig benetzen und dreidimensional frei quellen können, wobei aufgrund der Elastizität der Vliesmatrix dem Gelblocking vorgebeugt wird, da diese dem sich erhöhenden Platzbedarf bei Quellung des Superabsorbers folgen kann und dadurch die Partikel weitgehend voneinander separiert bleiben.

### 2. Verbesserter Tragekomfort

Durch die elastische Vliesstruktur, die einen hohen Anteil an Superabsorber enthält, ist es möglich, derzeit übliche, voluminöse SAP/Pulp-Mischungen zu ersetzen und die Nachteile herkömmlicher Inkontinenzartikel zu vermeiden. Die Dicke wird auf 1/10 reduziert. Durch die Anschmiegsamkeit, Elastizität und geringe Dicke des erfindungsgemäßen Materials sind dem Designer von Inkontinenzartikeln alle Möglichkeiten gegeben, wäscheähnlichen Tragekomfort mit Diskretion und technischer Performance zu vereinen.

### 3. Vereinfachter Herstellungsprozeß der Hygieneartikel

Darüber hinaus wird eine Vereinfachung des Windelherstellungsprozesses erreicht, indem einer Windelmaschine vorgeschaltete aufwändige Aufbereitungsanlagen für Pulp und Anlagen zur Mischung von SAP mit Pulp ersetzt werden durch Vorlage des elastischen, superabsorbierenden Vlieses in Form von Bahnen, Streifen oder Rollenware. Zusätzlich vereinfacht sich jegliche Windelkonstruktion, da auf Konstruktionselemente wie Tissue und Corewrap verzichtet werden kann. Der Windelaufbau reduziert sich auf z. B. eine elastische, atmungsaktive Membran, auf die der erfindungsgemäße Gegenstand (elastisches absorbierendes Vlies) beim Herstellungsprozeß aufgebracht wird, und die Abdeckung mit einem Verteilungs-und/oder Deckvlies. Dabei kann bereits beim Herstellungsprozeß des erfindungsgemäßen Materials dieses auf eine Membran oder Folie als Trägermaterial aufgebracht werden und im nachfolgenden Prozeßschritt mit herkömmlichen Vliesen abgedeckt werden. Im so erhaltenen Composite entspricht die Membran oder Folie herkömmlichem Backsheet, das erfindungsgemäße Material herkömmlichem Absorbent Core und das Abdeckvlies dem Distributionlayer bzw. Topsheet oder beiden bei geeigneter Konstruktion.

### 4. Geruch

Konventionelle Ansätze zur Geruchsbindung gehen davon aus, daß flüchtige, meist aminische Verbindungen als Geruchsträger durch bakterielle Zersetzung von Urininhaltsstoffen gebildet werden. Entsprechend wird durch Einstellung des Neutralisationsgrades der Superabsorber, durch Verwendung von pH-Puffern oder Bakteriostatika versucht, der Vermehrung und Aktivität von Mikroorganismen Einhalt zu gebieten. Dabei ist der Einsatz von Bakteriostatika oder gar Bakteriziden wegen der damit verbundenen Risiken, Allergien zu erzeugen, äußerst umstritten.

Basierend auf der Tatsache, daß Uringeruch aber im Wesentlichen der Geruch von Ammoniak-Gas ist, das sich durch enzymatische Zersetzung von Harnstoff bildet, das aktive Enzym zwar von Mikroorganismen zur Verstoffwechselung von Harnstoff benutzt wird, die Wirksamkeit und das Auftreten des Enzyms jedoch nicht an lebende Zellen gebunden ist, besteht der neuartige Ansatz zur Lösung dieses Problems konventioneller Inkontinenzartikel darin, nicht Mikroorganismen zu bekämpfen, sondern die Wirksamkeit harnstoffspaltender Enzyme durch spezifische Enzymblocker zu blockieren.

Der Ersatz herkömmlicher allergieerzeugender Zellgifte wie Bakterizide durch bekannte, unschädliche Enzymblocker beschränkt Techniken zur Geruchsbindung nicht nur auf Patienten bzw. Fälle, bei denen eine entsprechende Nutzen/Risiko-Bilanz vorliegt, sondern ermöglicht eine breite Anwendung zur Verbesserung der Lebensqualität von Inkontinenten.

Die Enzymblocker können bei der Herstellung des elastischen Vlieses der Spinnmasse beigemischt werden und nach Diffusion an der Oberfläche der Fasern wirksam sein, oder sie können nachträglich in Form einer Imprägnierung auf die Faseroberfläche appliziert werden.

### 5. Textile Flächengebilde

Aus der Literatur, z. B. aus dem US-Patent 4043331, ist bekannt, daß mit der Technologie des Electrospinning Polymere aus Lösung zu Vliesen aus Endlosfilamenten verarbeitet werden können. Das Electrospinning ist eine noch wenig verbreitete überwiegend experimentelle Technologie zur Erzeugung von Vliesstoffen. Es bietet jedoch die Möglichkeit, wie keine andere Vlieslegetechnik, Endlosfilamente aus verschiedenartigen Polymeren, auch Elastomeren, mit Durchmessern im Nanometer-Bereich zu erzeugen und in Form einer Art Spinnvlies abzulegen und während des Spinnprozesses Partikel wie z. B. Körner oder Mikrokapseln (gefüllt mit Wirkstoffen, Duftstoffen u. a., wasser- od. temperaturaktivierbar) in die Vliesstruktur mechanisch einzubinden. Zudem besteht die Möglichkeit, der Spinnlösung Wirkstoffe wie z. B. Enzyme, Enzymblocker, Vitamine, Tenside, Netzmittel u. a. zuzufügen, die infolge Diffusion an die Oberfläche der ersponnenen Fasern dort ihre Wirkung entfalten können.

L. M. Hansen et al. berichten in Journal of Applied Polymer Science, Vol. 95, pp. 427-434 (2005) über ein nach dem Verfahren des "Electrospinning" hergestelltes elastisches Vlies aus thermoplastischem Polyurethan, das mit modifizierter Stärke der Firma Grain Processing Corp., Muscatine, IA, mit der Handelsbezeichnung "Waterlock" als Superabsorber gefüllt ist. Dieses Material hat bereits interessante Eigenschaften, die jedoch nicht die Anforderungen in Bezug auf spezifische Absorption, Geschwindigkeit der Flüssigkeitsaufnahme und maximalen Füllgrad mit Absorber als Ersatz für ein Absorbent Core in Hygieneartikeln erfüllen.

In Erwartung, die Anforderungen bei Ersatz der modifizierten Stärke (Waterlock) des bestehenden Materials durch Polyacrylate, wie sie als Superabsorber in Hygieneartikeln üblich sind, erfüllen zu können, wurde handelsübliches Superabsorberpulver auf die für die Inkorporation in beschriebenes Nanovlies erforderlich erscheinende Korngröße zerkleinert und damit ein Superabsorber enthaltendes Nanovlies hergestellt. Erwartungsgemäß wurde zwar ein im Vergleich zu mit modifizierter Stärke (Waterlock) gefüllten Vliesen verbessertes Absorptionsverhalten festgestellt, jedoch erbrachten höhere Füllgrade als 50% keine meßbare Verbesserung der spezifischen Absorption infolge Gelblocking.

Überraschend wurde nun gefunden, daß Partikel von Superabsorber aus Natriumpolyacrylat geeigneter Korngröße, die nicht durch Zerkleinerung herkömmlicher Superabsorber-Pulver hergestellt wurden, sondern als Siebfraktion oberflächlich nachvernetzter Partikel gewonnen wurden, trotz im Vergleich zu herkömmlichem Superabsorber-Pulver grundsätzlich verzögerter Absorption dem Gesamtkonstrukt aus vorzugsweise Polyurethanvlies und Superabsorber deutlich überlegene Eigenschaften verleihen. Erst der Einsatz von oberflächlich nachvernetztem Superabsorber-Pulver aus Polyacrylat oder anderen geeigneten Copolymeren mit intakter Kern/Schale-Struktur und geeigneter Korngrößeverteilung ermöglicht schnelle Flüssigkeitsaufnahme und -verteilung und hohe spezifische Absorption bei gleichzeitig hohem Füllgrad von bis zu ca. 85% der aus thermoplastischem Polyurethan und Superabsorber-Pulver bestehenden superabsorbierenden Vliese mit Durchmessern der einzelnen Filamente im Nanometer- bis Micrometer-Bereich.

Die Erfindung wird nachfolgend anhand von Beispielen näher erläutert:

### Testmethoden

### A. Teabag Absorption Test (Tb)

Der "Teebeutel"-Absorptionstest gibt Aufschluß über die widerstandslose Flüssigkeitsabsorption. Eine definierte Menge einer SAP-Probe wird in einen handelsüblichen Teebeutel gefüllt; der Teebeutel wird 30 Minuten lang in eine überschüssige 0,9 %ige Kochsalzlösung getaucht, dann herausgenommen; danach läßt man den Beutel 10 Minuten lang abtropfen.

Der Tb-Wert in [g/g] ist das Verhältnis zwischen der absorbierten Wassermenge und der ursprünglichen SAP-Menge.

### B. Wasserretentionstest

Der Test zeigt die Wasserretention der aufgequollenen SAP-Probe. Der gequollene Teebeutel aus dem Tb-Test wird in eine Zentrifuge gegeben und bei einer Beschleunigung von 250 g 3 Minuten lang zentrifugiert.

Der CRC-Wert in [g/g] ist das Verhältnis zwischen der zurückbehaltenen Wassermenge und der ursprünglichen SAP-Menge.

### C. Vertikaler Dochteffekt

Der Test gibt Aufschluß über die Geschwindigkeit und die Vorzugsrichtung der Ausbreitung der Wasserabsorption. Ein 10x1 cm großes Klebeband mit darauf anhaftender SAP-Probe wird im Abstand von jeweils 1 cm markiert und senkrecht in eine 0,9 %ige NaCl-Lösung bis zur ersten 1 cm-Marke eingetaucht; die Flüssigkeitsausdehnung wird kompensiert durch Justieren des Streifens auf die Marke. Die Zeit, wann die Strecke von 1, 2, 3, 4 und 5 cm durch Aufsteigen der Lösung auf dem Band erreicht wird, wird gemessen.

### Ergebnisse

Es wurden Vliese aus thermoplastischem Polyurethan und verschiedenen absorbierenden Materialien mit verschiedenen Einsatzmengen nach dem Elektrospinnverfahren auf einer Laborspinnanlage bzw. Pilotspinnanlage hergestellt und den vorstehend beschriebenen Tests unterzogen.

In den erfindungsgemäßen Versuchen 3, 4, 6 und 7 kam als Superabsorber (SAP) oberflächlich vernetztes Natriumpolyacrylat-Pulver mit Kern/Mantel-Struktur zum Einsatz mit einer Korngrößenverteilung der Siebfraktion von d50 ca. 100µ und d100 ca. 150µ, Tb ca. 38 g/g und CRC ca. 22 g/g.

Vergleichsversuch 5 wurde mit mechanisch zerkleinertem (und deshalb nicht erfindungsgemäß) und danach gesiebtem Superabsorber (SAP) mit einer Korngrößenverteilung der Siebfraktion von d50 ca. 55µ und d100 von 100µ, Tb ca. 38 g/g, CRC ca. 22 g/g durchgeführt.

Kommerziell verfügbares SAP in Korngrößenfraktionen, wie sie heute in herkömmlichen Hygieneartikeln üblich sind, erwiesen sich als zu grob. Sie ließen sich nicht problemlos verarbeiten, waren nicht einwandfrei in die Vliesstruktur eingebunden und quollen bei Benetzung aus der Vliesstruktur heraus. Deshalb wurden bereits erste Vorversuche mit handelsüblichen SAP Typen eingestellt.

Die Ergebnisse der Versuche sind in Tabelle 1 zusammengefaßt.

Es zeigte sich, daß durch den Einsatz von oberflächlich vernetztem Natriumpolyacrylat-Pulver mit Kern/Mantel-Struktur bei Füllgraden um 50% und einer Korngröße von d50 = 100µ, d100 = 150µ die Geschwindigkeit der Wasseraufnahme von 450 sec. auf 80 sec. herabgesetzt werden konnte und die Wasseraufnahme von 29 g/g auf 40 g/g bzw. von 16 g/g auf 22 g/g gesteigert werden konnte. Bei 50 % Füllgrad ist noch kein Gelblocking festzustellen, vielmehr erreicht überraschend der Tb- wie auch der CRC-Wert die theoretischen Werte des reinen SAP. Ein auf einer größeren Pilotspinnanlage hergestelltes Muster gleicher Zusammensetzung zeigte vergleichbar gute Ergebnisse und bestätigt die Reproduzierbarkeit (Versuch 6).

Ferner zeigte sich in Versuch 5, daß sich eine Verletzung der nachvernetzten Schale der SAP-Partikel durch mechanische Zerkleinerung und eine damit einhergehende Vergrößerung des Anteils kleinerer Korngrößen in der Siebfraktion des SAP nachteilig auf die Geschwindigkeit der Flüssigkeitsaufnahme und Flüssigkeitsverteilung im Vlies auswirkt.

Aus den Versuchen zeigte sich auch, daß erst die Kombination von elastischer Vliesstruktur mit oberflächlich nachvernetztem SAP mit intakter Kern/Mantel-Struktur und geeigneter Korngröße zur besonders erwünschten Ausprägung der essentiellen Qualitätsmerkmale wie Geschwindigkeit der Flüssigkeitsaufnahme und -verteilung und Absorptionsvermögen (Tb, CRC) führt. Dabei wurde überraschend festgestellt, daß nicht ein besonders feines Pulver die beste Wirkung zeigt, wie aufgrund der feinen Vliesstruktur erwartet wurde, sondern daß es ein Optimum der Partikelgröße gibt, das deutlich grober ist als die Dimensionen der Vliesstrukturen.

**Tabelle 1:**

| **Absorbtionsverhalten verschiedener Vliesproben in Abhängigkeit vom Absorbermaterial und Füllgrad** | | | | | |
|---|---|---|---|---|---|
| **Nr.** | **Absorbermaterial** | **Füllgrad** | **Vertical Wicking 4 cm [sec]** | **Tb [g/g]** | **CRC [g/g]** |
| 1 | Waterlock | 50% | 450 | 29 | 16 |
| 2 | " | 70% | 480 | 24 | 14 |
| 3 | SAP (Kem/Mantet) | 40% | 260 | 41 | 22 |
| 4 | " | 50% | 80 | 40 | 18 |
| 5 | SAP (feineres Korn) | 50% | 170 | 41 | 21 |
| 6 | SAP (Kern/Mantel) (*) | 50% | 90 | 50 | 22 |
| 7 | SAP (Kern/Mantel) | 75% | 270 | 29 | 16 |

| | | | | | |
|---|---|---|---|---|---|
| (*) Pilotspinnanlage | | | | | |

## Patentansprüche

1. Superabsorberpulver, bestehend aus Polymerpartikeln, die einen in Gegenwart von Wasser aufquellenden Kern und eine oberflächlich nachvernetzte Schale aufweisen, wobei das Pulver eine Siebfraktion solcher Polymerpartikel ist, die nach der oberflächlichen Nachvernetzung ihrer Schale nicht zerkleinert worden sind, **dadurch gekennzeichnet, dass** die Siebfraktion eine Korngrößenverteilung von d50 = 55-100 µm und d100 = 100-150 µm aufweist.

2. Superabsorberpulver nach Anspruch 1, **dadurch gekennzeichnet, dass** das Polymer ein (Meth)Acrylat oder (Meth)Acryl-Copolymerisat ist.

3. Superabsorberpulver nach Anspruch 2, **dadurch gekennzeichnet, dass** das Polymer Natriumpolyacrylat ist.

4. Verwendung des Superabsorberpulvers gemäß einem der Ansprüche 1 bis 3 zur Ausrüstung von textilen Flächengebilden aus Feinstfasern oder - filamenten mit einem Durchmesser von weniger als 10 µm.

5. Verwendung nach Anspruch 4, **dadurch gekennzeichnet, daß** die Fasern oder Filamente einen Durchmesser von weniger als 1 µm besitzen.

6. Verwendung nach Anspruch 4 oder 5 zur Ausrüstung von Nanofaservliesen und daraus hergestellten Produkten.

7. Verwendung nach Anspruch 6 zur Ausrüstung von aus elektrostatisch gesponnenen Nanofasern hergestellten Produkten.

8. Verwendung nach einem der Ansprüche 4 bis 7, **dadurch gekennzeichnet, daß** die Fasern oder Filamente aus thermoplastischem und hydrophilem oder hydrophiliertem, schmelzspinnfähigem Polymerisat bestehen.

9. Verwendung nach Anspruch 8, **dadurch gekennzeichnet, daß** die Fasern oder Filamente aus Polyurethan bestehen.

10. Verwendung nach einem der Ansprüche 4 bis 9, **dadurch gekennzeichnet, daß** die textilen Flächengebilde Hygieneprodukte bilden.

11. Verwendung nach Anspruch 10 zur Ausrüstung von Inkontinenzprodukten, Damenbinden und Wundverbänden.

12. Verwendung eines mit einem Superabsorber gemäß einem der Ansprüche 1 bis 3 zur Absorption und Retention hydrophiler Fluide ausgerüsteten Nanofaservlieses zur Aufnahme und/oder verzögerten Abgabe mindestens eines der folgenden Fluide:
- Körperflüssigkeiten,
- Schweiß von Menschen und Tieren,
- Wasser, einschließlich Kühlwasser, Kondenswasser und Wasserdampf,
- Chemikalien, einschließlich Agrochemikalien und Pestiziden,
- Biozide, Germizide und Fungizide,
- Diagnostika,
- Brandschutz- und Feuerlöschmittel,
- Putz- und Reinigungsmittel,
- Hydraulikfluide,
- Heiz- und Kühlfluide,
- Abwässer, einschließlich radioaktiv kontaminierter Fluide,
- Parfümerien.

13. Verwendung des Nanofaservlieses nach Anspruch 12 als Inkontinenzartikel, Babywindel, Damenbinde, Kühlumschlag, Hygienetuch, Kosmetikpad oder Betteinlage oder als Teil der vorgenannten Gegenstände.

14. Verwendung des Nanofaservlieses nach Anspruch 12 als schweißaufsaugendes Einlagematerial für Schuhe, Bekleidungsstücke, Kopfbedeckungen, Stirnbänder, Handschuhe, Polstermöbel, Fahrzeugsitze, Sättel, Bettwäsche, Bettdecken, Pferdedecken, Sportartikel oder Sportgeräte.

15. Verwendung des Nanofaservlieses nach Anspruch 12 als Wischtuch, Aufnehmer, Unfall-Schutzmatte, Isomatte, Zeltplane, Feuchttuch, Trockentuch, Poliertuch, Putztuch oder Fensterleder oder als Teil der vorgenannten Gegenstände.

16. Verwendung des Nanofaservlieses nach Anspruch 12 als Boden- oder Wandbelag, Parkettunterspannbahn, Dachunterspannbahn, Brandschutzmatte, Leckageschutzmatte, Matte zur Auskleidung von Feuchträumen, Zelten, Fahrzeugen, Tanks oder Behältern oder als Teil der vorgenannten Gegenstände.

17. Verwendung des Nanofaservlieses nach Anspruch 12 als Filtermaterial, Verpackungsmaterial, Ummantelung, Dämmstoff oder Dichtmaterial oder als Teil der vorgenannten Gegenstände.

18. Verwendung des Nanofaservlieses nach Anspruch 12 als Geovlies, Drainagematte, Agrovlies oder Vlies zur verzögerten Abgabe von Chemikalien, Düngemitteln oder Pestiziden.

19. Verwendung nach einem der Ansprüche 12 bis 18, **dadurch gekennzeichnet, daß** das Vlies aus Feinstfasern oder -filamenten mit einem Durchmesser von weniger als 10 µm, vorzugsweise von weniger als 1 µm, besteht.

20. Verwendung nach einem der Ansprüche 12 bis 19, **dadurch gekennzeichnet, daß** das Vlies aus elektrostatisch gesponnenen Nanofasern hergestellt ist.

21. Verwendung nach einem der Ansprüche 12 bis 20, **dadurch gekennzeichnet, daß** die Fasern oder Filamente des Vlieses aus thermoplastischem und hydrophilem oder hydrophiliertem, schmelzspinnfähigem Polymerisat bestehen.

22. Verwendung nach Anspruch 21, **dadurch gekennzeichnet, daß** die Fasern oder Filamente aus Polyurethan bestehen.

23. Verwendung eines Superabsorberpulvers gemäß einem der Ansprüche 1 bis 3 bei der Herstellung eines mit dem Superabsorberpulver zur Absorption und Retention hydrophiler Fluide ausgerüsteten Nanofaservlieses zur Aufnahme und/oder verzögerten Abgabe mindestens eines der folgenden Fluide: Körperflüssigkeiten, Schweiß von Menschen und Tieren, Wasser, einschließlich Kühlwasser, Kondenswasser und Wasserdampf, Chemikalien, einschließlich Agrochemikalien und Pestiziden, Arzneimittel, Biozide, Germizide und Fungizide, Diagnostika, Brandschutz- und Feuerlöschmittel, Putz- und Reinigungsmittel, Hydraulikfluide, Heiz- und Kühlfluide, Abwässer, einschließlich radioaktiv kontaminierter Fluide, und Parfümerien.

## Claims

1. A superabsorbent powder consisting of polymer particles which have a core swelling in the presence of water and a superficially postcured shell, the powder being a screening fraction of such polymer particles which have not been crushed after the superficial postcure of their shell, **characterized in that** the screening fraction has a grain size distribution of d50 = 55 to 100 µm and d100 = 100 to 150 µm.

2. The superabsorbent powder according to claim 1, **characterized in that** the polymer is a (meth)acrylate or a (meth)acryl copolymer.

3. The superabsorbent powder according to claim 2, **characterized in that** the polymer is sodium polyacrylate.

4. Use of the superabsorbent powder according to any one of claims 1 to 3 for the finishing of textile webs of super-fine fibers or super-fine filaments having a diameter of less than 10 µm.

5. The use according to claim 4, **characterized in that** the fibers or filaments have a diameter of less than 1 µm.

6. The use according to claim 4 or 5 for the finishing of nanofiber nonwovens and products made thereof.

7. The use according to claim 6 for the finishing of products made from electrostatically spun nanofibers.

8. The use according to any one of claims 4 to 7, **characterized in that** the fibers or filaments consist of a thermoplastic and hydrophilic or hydrophilized polymer which is melt-spinnable.

9. The use according to claim 8, **characterized in that** the fibers or filaments consist of polyurethane.

10. The use according to any one of claims 4 to 9, **characterized in that** the textile webs constitute sanitary products.

11. The use according to claim 10 for the finishing of incontinence products, sanitary napkins and wound dressings.

12. Use of a nanofiber nonwoven, finished with a superabsorbent according to any one of claims 1 to 3 for the absorption and retention of hydrophilic fluids, for the uptake and/or sustained release of at least one of the following fluids:
- body fluids,
- sweat of humans and animals,
- water, including cooling water, condensation water and water vapor,
- chemicals, including agrochemicals and pesticides,
- biocides, germicides and fungicides,
- diagnostics,
- fire protection and fire extinguishing agents,
- cleaning agents,
- hydraulic fluids,
- heating and cooling fluids,
- sewage, including radioactively contaminated fluids,
- perfumes.

13. The use of the nanofiber nonwoven according to claim 12 as an incontinence article, baby diaper, sanitary napkin, cooling compress, sanitary tissue, cosmetic pad or bed inlay or as a part of the aforementioned goods.

14. The use of the nanofiber nonwoven according to claim 12 as a sweat absorbing padding material for shoes, garments, headgears, headbands, gloves, upholstered furniture, vehicle seats, saddles, bed linen, bedspreads, horse blankets, sports goods or pieces of sports equipment.

15. The use of the nanofiber nonwoven according to claim 12 as a wipe, floor cloth, casualty protection mat, camping mat, tarpaulin, wet cloth, drying cloth, polishing cloth, cleaning cloth or washleather or as a part of the aforementioned goods.

16. The use of the nanofiber nonwoven according to claim 12 as a floor covering or wall covering, parquet underlay sheeting, roof underlay sheeting, fire protection mat, leakage protection mat, mat for the lining of damp rooms, tents, vehicles, tanks or containers or as a part of the aforementioned goods.

17. The use of the nanofiber nonwoven according to claim 12 as a filter material, packaging material, sheathing, insulating material or sealing material or as a part of the aforementioned goods.

18. The use of the nanofiber nonwoven according to claim 12 as a geo web, drainage mat, agro web or nonwoven for the sustained release of chemicals, fertilizers or pesticides.

19. The use according to any one of claims 12 to 18, **characterized in that** the nonwoven consists of super-fine fibers or filaments having a diameter of less than 10 µm, preferably of less than 1 µm.

20. The use according to any one of claims 12 to 19, **characterized in that** the nonwoven is manufactured from electrostatically spun nanofibers.

21. The use according to any one of claims 12 to 20, **characterized in that** the fibers or filaments of the nonwoven consist of a thermoplastic and hydrophilic or hydrophilized polymer which is melt-spinnable.

22. The use according to claim 21, **characterized in that** the fibers or filaments consist of polyurethane.

23. Use of a superabsorbent powder according to any one of claims 1 to 3 in the manufacture of a nanofiber nonwoven, finished with the superabsorbent powder for the absorption and retention of hydrophilic fluids, for the uptake and/or sustained release of at least one of the following fluids:
body fluids, sweat of humans and animals, water, including cooling water, condensation water and water vapor, chemicals, including agrochemicals and pesticides, pharmaceuticals, biocides, germicides and fungicides, diagnostics, fire protection and fire extinguishing agents, cleaning agents, hydraulic fluids, heating and cooling fluids, sewage, including radioactively contaminated fluids, and perfumes.

## Revendications

1. Poudre super-absorbante, constituée de particules polymères qui présentent un noyau gonflant en présence d'eau et une enveloppe post-réticulée superficiellement, la poudre étant une fraction de tamisage du type de particules polymères qui n'ont pas été broyées après la post-réticulation superficielle de leur enveloppe, **caractérisée en ce que** la fraction de tamisage présente une distribution granulométrique de d50 = 55-100 µm et d100 = 100-150 µm.

2. Poudre super-absorbante selon la revendication 1, **caractérisée en ce que** le polymère est un copolymère de (méth)acrylate ou un copolymère (méth)acrylique.

3. Poudre super-absorbante selon la revendication 2, **caractérisée en ce que** le polymère est du polyacrylate de sodium.

4. Utilisation de la poudre super-absorbante selon l'une des revendications 1 à 3 pour l'équipement de surfaces planes textiles en fibres très fines ou en filaments très fins d'un diamètre inférieur à 10 µm.

5. Utilisation selon la revendication 4, **caractérisée en ce que** les fibres ou filaments présentent un diamètre inférieur à 1 µm.

6. Utilisation selon la revendication 4 ou 5 pour l'équipement de non-tissés de nanofibres et de produits fabriqués à partir de ceux-ci.

7. Utilisation selon la revendication 6 pour l'équipement de produits fabriqués de nanofibres filées électrostatiquement.

8. Utilisation selon l'une des revendications 4 à 7, **caractérisée en ce que** les fibres ou filaments sont en polymère thermoplastique et hydrophile ou hydrophilisé et apte à être filé à chaud.

9. Utilisation selon la revendication 8, **caractérisée en ce que** les fibres ou les filaments sont en polyuréthane.

10. Utilisation selon l'une des revendications 4 à 9, **caractérisée en ce que** les surfaces planes textiles forment des produits hygiéniques.

11. Utilisation selon la revendication 10 pour l'équipement de produits d'incontinence, de serviettes hygiéniques ou de pansements.

12. Utilisation d'un non-tissé de nanofibres équipé d'un super-absorbant selon l'une des revendications 1 à 3 pour l'absorption et la rétention de fluides hydrophiles pour la réception et/ou la libération retardée d'au moins un des fluides suivants :
- liquides biologiques,
- sueur humaine et animale,
- eau, y compris eau de refroidissement, eau de condensation et vapeur d'eau,
- produits chimiques, y compris produits agrochimiques et pesticides,
- produits biocides, germicides et fongicides,
- produits de diagnostic,
- agents ignifuges et extincteurs,
- agents de nettoyage et détergents,
- fluides hydrauliques,
- fluides de chauffage et de refroidissement,
- eaux usées, y compris les fluides contaminés par radioactivité,
- produits de parfumerie.

13. Utilisation du non-tissé de nanofibres selon la revendication 12 comme article d'incontinence, couche pour bébé, serviette hygiénique, compresse de froid, lingette hygiénique, disque démaquillant ou alèse de lit, ou comme élément de l'un des produits cités ci-dessus.

14. Utilisation du non-tissé de nanofibres selon la revendication 12 comme matière de rembourrage absorbant la sueur pour des chaussures, des vêtements, des couvre-chefs, des bandeaux, des gants, des meubles rembourrés, des sièges de voiture, des selles, des parures de lit, des couvertures, des couvertures pour chevaux, des articles de sport ou des appareils de sport.

15. Utilisation du non-tissé de nanofibres selon la revendication 12 comme torchon, serpillère, natte de protection en cas d'accident, tapis de sol, toile de tente, lingette humide, lingette sèche, tissu de polissage, chiffon ou peau de chamois, ou comme élément des produits cités ci-dessus.

16. Utilisation du non-tissé de nanofibres selon la revendication 12 comme revêtement de sol ou mural, sous-couche de parquet, écran de sous-toiture, tapis anti-feu, tapis de protection contre les fuites, natte pour le revêtement de locaux humides, de tentes, de véhicules, de citernes ou de réservoirs, ou comme élément des produits cités ci-dessus.

17. Utilisation du non-tissé de nanofibres selon la revendication 12 comme matériau filtrant, matériau d'emballage, enveloppe, matériau isolant ou matériau d'étanchéité, ou comme élément des produits cités ci-dessus.

18. Utilisation du non-tissé de nanofibres selon la revendication 12 comme géo-non-tissé, tapis de drainage, agro-non-tissé ou non-tissé pour la libération retardée de produits chimiques, d'engrais ou de pesticides.

19. Utilisation selon l'une des revendications 12 à 18, **caractérisée en ce que** le non-tissé est composé de fibres très fines ou de filaments très fins d'un diamètre inférieur à 10 µm, de préférence inférieur à 1 µm.

20. Utilisation selon l'une des revendications 12 à 19, **caractérisée en ce que** le non-tissé est fabriqué à partir de nanofibres filées électrostatiquement.

21. Utilisation selon l'une des revendications 12 à 20, **caractérisée en ce que** les fibres ou filaments du non-tissé sont en polymère thermoplastique et hydrophile ou hydrophilisé et apte à être filé à chaud.

22. Utilisation selon la revendication 21, **caractérisée en ce que** les fibres ou les filaments sont en polyuréthane.

23. Utilisation d'une poudre super-absorbante selon l'une des revendications 1 à 3 pour la fabrication d'un non-tissé de nanofibres équipé de la poudre super-absorbante pour l'absorption et la rétention de fluides hydrophiles pour la réception et/ou la libération retardée d'au moins un des fluides suivants :
liquides biologiques, sueur humaine et animale, eau, y compris eau de refroidissement, eau de condensation et vapeur d'eau, produits chimiques, y compris produits agrochimiques et pesticides, médicaments, produits biocides, germicides et fongicides, produits de diagnostic, agents ignifuges et extincteurs, agents de nettoyage et détergents, fluides hydrauliques, fluides de chauffage et de refroidissement, eaux usées, y compris les fluides contaminés par radioactivité, produits de parfumerie.
